(19) Europäisches Patentamt
European Patent Office
Office européen des brevets

(11) **EP 4 595 891 A1**

(12) **EUROPEAN PATENT APPLICATION**

(43) Date of publication:
06.08.2025 Bulletin 2025/32

(21) Application number: 25154820.2

(22) Date of filing: 29.01.2025

(51) International Patent Classification (IPC):
*A61B 6/03* (2006.01)     *A61B 6/00* (2024.01)
*A61B 6/50* (2024.01)

(52) Cooperative Patent Classification (CPC):
**A61B 6/032; A61B 6/486; A61B 6/504;
A61B 6/507; A61B 6/5217**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **02.02.2024 JP 2024014843**

(71) Applicant: **Canon Medical Systems Corporation
Tochigi 324-0036 (JP)**

(72) Inventors:
• **ARAKAKI, Ryuichi
Otawara-shi, 324-0036 (JP)**
• **SHIROISHI, Ryo
Otawara-shi, 324-0036 (JP)**

(74) Representative: **Marks & Clerk LLP
15 Fetter Lane
London EC4A 1BW (GB)**

(54) **MEDICAL IMAGE PROCESSING DEVICE, MEDICAL IMAGE PROCESSING METHOD, AND PROGRAM**

(57) A medical image processing device of an embodiment includes an acquirer, a deriver, and an analyzer. The acquirer is configured to acquire medical images of a plurality of time phases of a brain of a subject, having one of left and right sides of the brain as an affected side and the other as a healthy side. The deriver is configured to derive feature amounts related to collateral circulation in the brain of the subject based on the acquired original medical images. The analyzer is configured to analyze a time phase delay in the affected side relative to the healthy side based on a result of comparing the feature amounts of the affected side and the healthy side for each of the plurality of time phases.

FIG. 2

MEDICAL IMAGE PROCESSING DEVICE ~100

PROCESSING CIRCUITRY ~140
ACQUISITION FUNCTION ~141
IDENTIFICATION FUNCTION ~142
GENERATION FUNCTION ~143
DERIVATION FUNCTION ~144
ANALYSIS FUNCTION ~145
DISPLAY CONTROL FUNCTION ~146

COMMUNICATION INTERFACE ~110
INPUT INTERFACE ~120
DISPLAY ~130

MEMORY ~150

EP 4 595 891 A1

**Description**

FIELD

The embodiments disclosed in this specification and drawings relate to a medical image processing device, a medical image processing method, and a program.

BACKGROUND

As technology for diagnosing cerebral infarction, for example, there is technology of analyzing computed tomography (CT) images and quantitatively measuring the state of collateral circulation. In this technology, a quantitative index of collateral circulation is calculated from a vascular control area and an area of interest analyzed using 4D-CT Angiography (CTA), and the calculated quantitative index is used to diagnose cerebral infarction. Collateral circulation is a circulatory blood vessel that is newly generated to compensate for deterioration of blood flow when stenosis or occlusion has occurred.

Alternatively, there is technology of dividing the entire brain into hemispheres and using CT images of a side with cerebral infarction symptoms (hereinafter, an affected side) and a side without symptoms (hereinafter, a healthy side). This technique counts the number of pixels in areas filled with blood vessels on both the affected side and the healthy side, obtains the ratio between the total amounts of blood vessel filling on the affected side and the healthy side (hereafter referred to as a left-right ratio), and uses the same as a quantitative index of collateral circulation.

The technology using 4D-CTA has the advantage that it allows detailed analysis including delays in blood flow arrival time. However, imaging needs to be performed approximately 20 times per minute in order to obtain a CT image, which has the disadvantages of long imaging time and high radiation exposure during imaging.

On the other hand, the technology for obtaining the left-right ratio has the advantage that analysis can be performed with one-time imaging. However, although information on the proportion of blood vessels is used, there is a difference in blood flow arrival time between the affected side and the healthy side, which makes the analysis insufficient and makes it difficult to accurately evaluate the condition of a disease, such as collateral circulation.

An object of embodiments disclosed in this specification and drawings is to enable accurate evaluation of the condition of a disease. However, the object of the embodiments disclosed in this specification and drawings is not limited to the above object. Objects corresponding to the effects of each configuration shown in embodiments which will be described later can also be positioned as other objects.

A medical image processing device of an embodiment includes an acquirer, a deriver, and an analyzer. The acquirer is configured to acquire medical images of a plurality of time phases of the brain of a subject, having one of left and right sides of the brain as an affected side and the other as a healthy side. The deriver is configured to derive feature amounts related to collateral circulation in the brain of the subject based on the acquired original medical images. The analyzer is configured to analyze a time phase delay in the affected side relative to the healthy side based on a result of comparing the feature amounts of the affected side and the healthy side for each of the plurality of time phases.

The medical image processing device may further include a display controller configured to cause a display to display delay information regarding the determined time phase delay.

In the medical image processing device, the deriver may be configured to derive a ratio between inflow amounts of fluid flowing into blood vessels on the affected side and the healthy side for each of the plurality of time phases, and the analyzer may be configured to analyze the time phase delay based on the ratio between the inflow amounts.

In the medical image processing device, the deriver may be configured to derive a ratio between presence of blood vessels on the affected side and the healthy side for each of the plurality of time phases, and the analyzer may be configured to analyze the time phase delay based on the ratio between the presence.

In the medical image processing device, the deriver may be configured to derive a ratio between outflow amounts of fluid flowing out of blood vessels on the affected side and the healthy side for each of the plurality of time phases, and the analyzer may be configured to analyze the time phase delay based on the ratio between the outflow amounts.

In the medical image processing device, the analyzer may be configured to determine that there is a time phase delay when the ratio between the inflow amounts is less than a first threshold value.

In the medical image processing device, the analyzer may be configured to determine that there is the time phase delay when the ratio between the presence is less than a second threshold value.

In the medical image processing device, the analyzer may be configured to determine that there is a time phase delay when the ratio between the outflow amounts is equal to or greater than a third threshold value.

In the medical image processing device, the deriver may be configured to derive an affected-side collateral circulation time ratio by comparing blood vessel presence ratios in different time phases on the affected side, and the analyzer may be configured to determine the time phase delay based on the affected-side collateral circulation time ratio.

The medical image processing device may further include a generator configured to perform maximum intensity projection processing on the original medical images to generate a maximum intensity projection image. The deriver may be configured to derive the feature amounts based on the maximum intensity projection image.

The medical image processing device may further include an identifier configured to identify a projection processing range that is a target of the maximum intensity projection processing.

The medical image processing device may further include a receiver configured to receive identification of the projection processing range by a user.

In the medical image processing device, the analyzer may be configured to analyze the time phase delay based on an amount of change over time in a total vascular filling amount on the affected side.

In the medical image processing device, the generator may be configured to generate vascular images based on the maximum intensity projection image, and the vascular images in different time phases may be superimposed and displayed on a display.

In the medical image processing device, the maximum intensity projection image may be an image including at least one of an entire region, an anterior cerebral artery region, a middle cerebral artery region, and a posterior cerebral artery region in the brain.

A medical image processing method of another embodiment includes, using a computer, acquiring original medical images of a plurality of time phases of a brain of a subject, with one of left and right sides of the brain being an affected side and the other being a healthy side, deriving feature amounts related to collateral circulation in the brain of the subject based on the acquired original medical images, and analyzing a time phase delay in the affected side relative to the healthy side based on a result of comparing the feature amounts of the affected side and the healthy side for each of the plurality of time phases.

A program of another embodiment causes a computer to acquire original medical images of a plurality of time phases of a brain of a subject, with one of left and right sides of the brain being an affected side and the other being a healthy side, derive feature amounts related to collateral circulation in the brain of the subject based on the acquired original medical images, and analyze a time phase delay in the affected side relative to the healthy side based on a result of comparing the feature amounts of the affected side and the healthy side for each of the plurality of time phases.

BRIEF DESCRIPTION OF THE DRAWINGS

FIG. 1 is a block diagram showing an example of a configuration of an in-hospital system 1 of a first embodiment.
FIG. 2 is a block diagram showing an example of a configuration of a medical image processing device 100 of the first embodiment.
FIG. 3 is a flowchart showing an example of processing in the medical image processing device 100.
FIG. 4 is a diagram showing an example of change over time in a sum value of the number of blood vessel pixels on each of an affected side and a healthy side.
FIG. 5 is a diagram showing an example of change over time in the sum value of the number of blood vessel pixels on each of the affected side and the healthy side.
FIG. 6 is a diagram showing an example of change over time in the sum value of the number of blood vessel pixels on each of the affected side and the healthy side.
FIG. 7 is a diagram showing an example of images of a procedure for combining images for two time phases.
FIG. 8 is a diagram showing an example of images of a procedure for combining images for one time phase and two time phases.
FIG. 9 is a block diagram showing an example of a configuration of a medical image processing device 200 of the second embodiment.
FIG. 10 is a diagram showing an example of change over time in a sum value of the number of blood vessel pixels on each of an affected side and a healthy side.

DETAILED DESCRIPTION

Hereinafter, a medical image processing device, a medical image processing method, and a program according to embodiments will be described with reference to the drawings.

For cerebral infarction patients, the presence of collateral circulation is an indicator of the possibility of tissue recovery, and thus evaluation thereof is important. In clinical practice, for example, doctors visually evaluate the degree of development of collateral circulation from blood vessel images visualized by CT Angiography (CTA) or Magnetic Resonance Angiography (MRA), and use the same as a basis for making treatment decisions for patients.

Specifically, an affected side where the infarction has occurred is compared with a healthy side on the opposite side, and the state of collateral circulation is qualitatively evaluated by visually checking the proportion of blood vessels that can be confirmed on the affected side compared to the healthy side. Alternatively, in addition thereto, the state of collateral circulation is qualitatively evaluated by visually checking the degree of delay in blood flow arrival time on the affected side compared to the healthy side.

However, since these are qualitative evaluations, it is difficult for treating doctors to agree on precise determination. Therefore, a medical image processing device of an embodiment analyzes CT images to quantitatively measure the state of collateral circulation. Furthermore, the medical image processing device of the embodiment calculates a left-right ratio between the healthy side and the affected side in consideration of a delay in blood flow arrival time (time phase delay), thereby enabling accurate evaluation of the condition of a disease, such as collateral circulation.

(First embodiment)

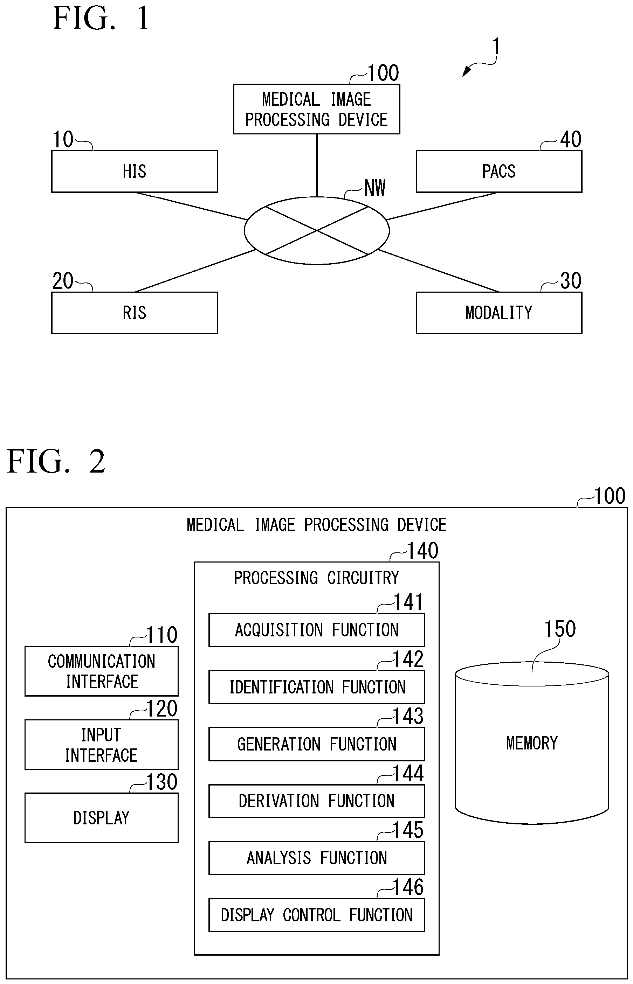

FIG. 1 is a block diagram showing an example of a configuration of an in-hospital system 1 according to a first embodiment. The in-hospital system 1 according to the first embodiment includes, for example, a hospital information system (hereinafter, HIS) 10, a radiology information system (hereinafter, RIS) 20, a medical image diagnostic device (modality) 30, a picture archiving and communication system (PACS) 40, and a medical image processing device 100.

The HIS 10 is a computer system that supports operations within a hospital. Specifically, the HIS 10 has various subsystems. The various subsystems include, for example, an electronic medical record system, a medical accounting system, a medical appointment system, a hospital reception system, and an admission and discharge management system.

The HIS 10 includes, for example, a computer such as a server device or a client terminal equipped with a processor such as a central processing unit (CPU), a memory such as a read only memory (ROM) and a random access memory (RAM), a display, an input interface, and a communication interface.

A user inputs and refers to information on a patient using an electronic medical record system included in the HIS 10. The user issues an order for an imaging examination to the HIS 10. The HIS 10 transfers order information corresponding to the imaging examination order to other systems such as the RIS 20.

The RIS 20 is a computer system that supports operations in an imaging diagnosis department. The RIS 20 performs management of reservations for imaging examination orders in cooperation with the HIS 10, linkage reservation information to examination equipment, management of examination information, and the like. The RIS 20 includes a computer such as a server device or a client terminal including, for example, a processor such as a CPU, a memory such as a ROM and a RAM, a display, an input interface, and a communication interface.

The modality 30 performs imaging (photographing) according to imaging conditions (photography protocols) determined on the basis of, for example, imaging examination instructions. Examples of the modality 30 include an X-ray computed tomography device, an X-ray diagnostic device, a magnetic resonance imaging device, an ultrasound diagnostic device, and a nuclear medicine diagnostic device. The modality 30 generates medical images (original medical images) on the basis of an operation of a user such as a doctor (radiologist) or a diagnostic radiologist. In the embodiment, the modality 30 is a CT device, and the original medical images are four-dimensional CT images obtained by dynamic imaging of a subject. The generated four-dimensional CT images are transmitted to the PACS 40. The modality 30 is an example of a medical image generation device.

The PACS 40 is a computer system that receives four-dimensional CT images transmitted by the modality 30 and medical images transmitted by other external devices and stores the same in a database. In response to a request from a client, the PACS 40 transmits (transfers) medical images such as four-dimensional CT images stored in the database. The PACS 40 includes a server computer including a processor such as a CPU, a memory such as a ROM and a RAM, a display, an input interface, and a communication interface.

The configuration of the in-hospital system 1 is not limited to the above. The in-hospital system 1 may include, for example, an image interpretation report creation device. In addition, some elements of the in-hospital system 1 may be integrated. For example, the HIS 10 and the RIS 20 may be integrated into one system.

The medical image processing device 100 is a device that provides information for a doctor to diagnose cerebral infarction in a subject by image processing four-dimensional CT images of a plurality of time phases. The medical image processing device 100 generates a maximum intensity projection image (hereinafter, MIP image) by, for example, performing maximum intensity projection (MIP) processing on a four-dimensional CT image. The medical image processing device 100 generates an MIP image by, for example, identifying a projection processing range (hereinafter, MIP range) on the four-dimensional CT image and performing MIP processing on the MIP range.

FIG. 2 is a block diagram showing an example of a configuration of the medical image processing device 100 of the first embodiment. The medical image processing device 100 includes, for example, a communication interface 110, an input interface 120, a display 130, processing circuitry 140, and a memory 150. The communication interface 110, the input interface 120, and the display 130 in the medical image processing device 100 are provided separately from a communication interface, an input interface, and a display provided in the HIS 10, but these components may be common.

The communication interface 110 communicates with external devices such as the RIS 20, the modality 30, and the PACS 40 via a network NW such as a local area network (LAN). The communication interface 110 includes, for example, a communication interface such as a network interface card (NIC). The network NW may include the Internet, a cellular network, a Wi-Fi network, a wide area network (WAN), and the like instead of or in addition to the LAN.

The input interface 120 receives various input operations from a user such as a medical practitioner, converts the received input operations into electrical signals, and outputs the electrical signals to the processing circuitry 140. For example, when an input operation is performed by the user, the input interface 120 generates information corresponding to the input operation. The input interface 120 outputs the generated information corresponding to the input operation to the processing circuitry 140.

The input interface 120 includes, for example, a mouse, a keyboard, a trackball, a switch, a button, a joystick, a touch panel, and the like. The input interface 120 may be, for example, a user interface that receives audio input, such as a microphone. If the input interface 120 is a touch panel, the input interface 120 may also have the display function of the display 130.

In this specification, the input interface is not limited to only those that have physical operating parts such as a mouse and a keyboard. For example, electrical signal processing circuitry that receives an electrical signal corresponding to an input operation from an external input apparatus provided separately from the device and outputs the electrical signal to a control circuit is also included as an example of the input interface.

The display 130 displays various types of information. For example, the display 130 displays images generated by the processing circuitry 140, a graphical user interface (GUI) for receiving various input operations from a user, and the like. For example, the display 130 is a liquid crystal display (LCD), a cathode ray tube (CRT) display, an organic electroluminescence (EL) display, or the like. The display 130 is an example of a display.

The processing circuitry 140 includes, for example, an acquisition function 141, an identification function 142, a generation function 143, a derivation function 144, an analysis function 145, and a display control function 146. The processing circuitry 140 realizes these functions by, for example, a hardware processor (computer) executing a program stored in a memory (storage circuit) 150.

The hardware processor is, for example, circuitry such as a CPU, a graphics processing unit (GPU), an application specific integrated circuit (ASIC), a programmable logic device (for example, a simple programmable logic device (SPLD) or a complex programmable logic device (CPLD)), a field programmable gate array (FPGA), or the like.

Instead of storing the program in the memory 150, the program may be directly embedded in the circuit of the hardware processor. In this case, the hardware processor realizes the functions by reading and executing the program embedded in the circuit. The aforementioned program may be stored in the memory 150 in advance, or may be stored in a non-transient storage medium such as a DVD or a CD-ROM and installed in the memory 150 from the non-transient storage medium by setting the non-transient storage medium in a drive device (not shown) of the medical image processing device 100.

The hardware processor is not limited to being configured as a single circuit, but may be configured as one hardware processor by combining a plurality of independent circuits to realize each function. In addition, a plurality of components may be integrated into one hardware processor to realize each function. The hardware processor, the memory, and the like in the medical image processing device 100 are provided separately from the hardware processor, the memory, and the like of the HIS 10, but the components may be common.

The memory 150 is realized by, for example, a semiconductor memory element such as a RAM or a flash memory, a hard disk, or an optical disc. Such non-transient storage media may be realized by other storage devices connected via a communication network, such as a network attached storage (NAS) or an external storage server device. The memory 150 may include a non-transient storage medium such as a read only memory (ROM) or a register.

The acquisition function 141 acquires medical images of a plurality of time phases of the brain, having one of the left and right sides of the brain of a subject as an affected side and the other side as a healthy side. The acquisition function 141 is an example of an acquisition function. The acquisition function 141 acquires medical images provided by, for example, the modality 30 or the PACS 40. The acquisition function 141 is an example of an acquirer.

The identification function 142 identifies an MIP range in a 4D CT image. For example, the identification function 142 identifies an MIP range on each of the affected side and the healthy side in a 4D CT image acquired by the acquisition function 141. The identification function 142 identifies an MIP range, for example, on the basis of information such as a location where infarction is assumed to have occurred. The identification function 142 may use the entire brain as an MIP range or may use a specific range in the brain as an MIP range.

The generation function 143 generates an MIP image as a medical image on the basis of a 4D CT image generated by the modality 30. The generation function 143 generates MIP images of the affected side and the healthy side as MIP images, for example, by MIP processing the MIP range of the 4D CT image identified by the identification function 142 on each of the affected side and the healthy side.

The generation function 143 generates an MIP image for each of four-dimensional CT images of a plurality of time phases. The generation function 143 further generates a vascular image showing blood vessels including collateral circulation for each of the affected side and the healthy side on the basis of the generated MIP images. The generation function 143 is an example of a generator.

The derivation function 144 derives feature amounts related to collateral circulation in the brain of the subject on the basis of the MIP images generated by the generation function 143. The derivation function 144 calculates the total vascular filling amount in each of the affected side and the healthy side of the brain by, for example, counting the number of pixels in an area filled with blood vessels (hereinafter, the number of blood vessel pixels) in the MIP image of each of the affected side and the healthy side of the brain. The derivation function 144 derives feature amounts on the basis of the calculated total vascular filling amount. The derivation function 144 derives feature amounts of blood vessels in a plurality of time phases. The derivation function 144 is an example of a deriver.

The analysis function 145 compares the feature amounts on the affected side and the healthy side for each of the plurality of time phases. The feature amounts include, for example, a left/right inflow ratio, a left/right presence ratio, and a left/right outflow ratio. The analysis function 145 analyzes a delay in a time phase of the affected side relative to the healthy side on the basis of the result of comparing the feature amounts on the affected side and the healthy side. The analysis function 145 is an example of an analyzer.

The left/right inflow ratio is the ratio of the amount of fluid (blood and contrast agent) that flows into blood vessels (including collateral circulation) on the affected side and the healthy side. The left/right presence ratio is the ratio of the amount of blood vessels present on the affected side and the healthy side. The left/right outflow ratio is the ratio of the amount of fluid that flows out of blood vessels on the affected side and the healthy side.

The analysis function 145 determines a delay in the time phase of the affected side relative to the healthy side on the basis of the relationship between a left/right ratio, such as the left/right inflow ratio, the left/right outflow ratio, or the left/right presence ratio, and a predetermined threshold value. The analysis function 145 determines that there is a time phase delay, for example, when the left/right inflow ratio is less than a first threshold value. The analysis function 145 determines that there is a time phase delay between different time phases, for example, when the left/right presence ratio between different time phases is less than a second threshold value. The analysis function 145 determines that there is a time phase delay, for example, when the left/right outflow ratio is equal to or greater than a third threshold value.

The analysis function 145 may generate side-specific fluid volume information by comparing fluid volume information between different time phases on the affected side and the healthy side, for example. The analysis function 145 may determine a time phase delay regarding the fluid volume on the affected side relative to the healthy side on the basis of the result of comparison between the side-specific fluid volume information of the affected side (hereinafter, affected side individual fluid volume information) and the side-specific fluid volume information of the healthy side (hereinafter, healthy side individual fluid volume information).

The display control function 146 causes the display 130 to display various images. The display control function 146 causes the display 130 to display, for example, a four-dimensional CT image acquired by the acquisition function 141, an MIP image generated by the generation function 143, and delay information regarding a time phase delay, such as, the determination result in the analysis function 145. The display control function 146 is an example of a display controller.

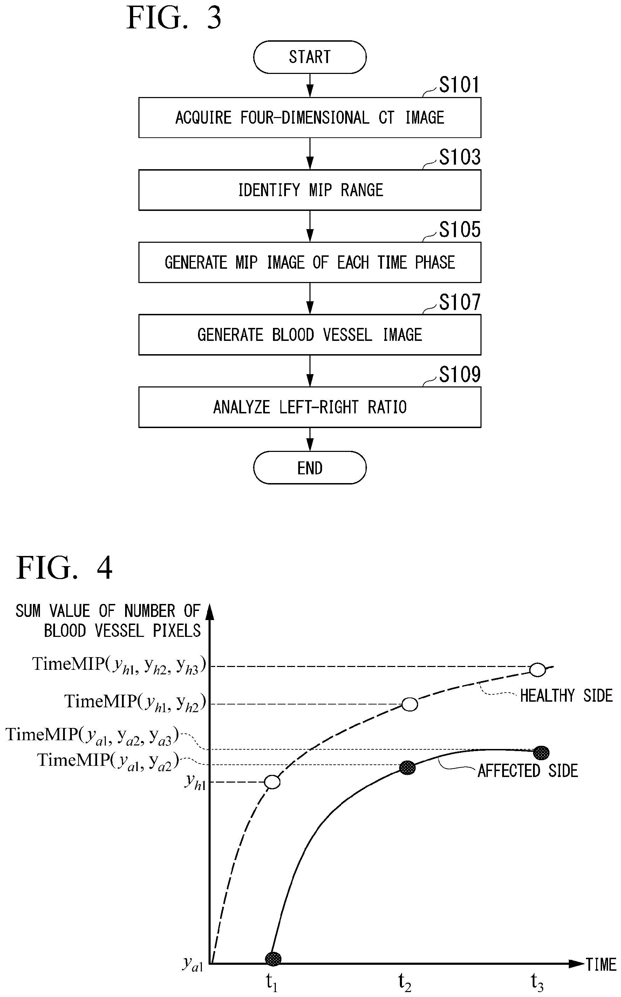

Next, processing in the medical image processing device 100 of the first embodiment will be described. FIG. 3 is a flowchart showing an example of processing in the medical image processing device 100. First, the medical image processing device 100 acquires four-dimensional CT images of a plurality of time phases stored in the PACS 40 through the acquisition function 141 (step S101).

Subsequently, the identification function 142 identifies an MIP range in the four-dimensional CT images of the plurality of time phases on each of the affected side and the healthy side (step S103). In identifying the MIP range, the identification function 142 may, for example, set the entire brain region (whole brain), the anterior cerebral artery region (ACA region), the middle cerebral artery region (MCA region), or the posterior cerebral artery region (PCA region) as the MIP range, or may set a plurality of these regions or other ranges as the MIP range.

Subsequently, the generation function 143 performs MIP processing on the MIP range in the four-dimensional CT images identified by the identification function 142 to generates an MIP image (step S105). The generation function 143 generates an MIP image in the MIP range on each of the affected side and the healthy side in each of the four-dimensional CT images of the plurality of time phases.

Subsequently, the generation function 143 generates vascular images on the affected side and the healthy side of the brain on the basis of the generated MIP images (step S107). For the vascular images, the generation function 143 generates vascular images that represent the blood vessels within the MIP range on each of the affected side and the healthy side in each of the MIP images of the plurality of time phases.

Subsequently, the derivation function 144 and the analysis function 145 analyze the left/right ratio of the affected side and the healthy sides (step S109). In analyzing the left/right ratio, the derivation function 144 derives a feature amount on each of the affected side and the healthy side on the basis of the MIP images generated by the generation function 143. The derivation function 144 derives, for example, any of the left/right inflow ratio, the left/right presence ratio, and the left/right outflow ratio as the feature amount. Hereinafter, analysis of each of the left/right inflow ratio, left/right presence ratio, and left/right outflow ratio will be described.

The derivation function 144 calculates the left/right inflow ratio Output(1) at a specific timing by putting the number of blood vessel pixels in a plurality of time phases into the following formula (1). The number of pixels ya1 in Formula (1) is the number of affected-side blood vessel pixels at a time t1 and represents the total filling amount of blood vessels in the affected-side hemisphere at the time 11. The number of pixels ya1 can be represented, for example, by the following formula (2). Change over time in the sum value of the number of blood vessel pixels on each of the affected side and the healthy side at this time is shown as a graph in FIG. 4. FIG. 4 is a diagram showing an example of change over time in the sum value of the number of blood vessel pixels on each of the affected side and the healthy side.

[Expression 1]

$$\text{Output}(1) = \frac{ya1 + TimeMIP(ya1,ya2) + TimeMIP(ya1,ya2,ya3)}{yh1 + TimeMIP(yh1,yh2) + TimeMIP(yh1,yh2,yh3)} \quad \cdots (1)$$

ya1: Number of affected-side blood vessel pixels (time t1)
TimeMIP(ya1,ya2): Sum value of number of affected-side blood vessel pixels (time t1 and t2)
TimeMIP(ya1,ya2,ya3): Sum value of number of affected-side blood vessel pixels (time t1, t2, and t3)
yh1: Number of healthy-side blood vessel pixels (time t1)
TimeMIP(yh1,yh2): Sum value of number of healthy-side blood vessel pixels (time t1, t2)
TimeMIP(yh1,yh2,yh3): Sum value of number of healthy-side blood vessel pixels (time t1, t2, and t3)

$$y_{a1} = \sum_{i=0}^{N_v - 1} m_a(i) G(\text{I}(\text{I,t})) \cdots (2)$$

$N_v$: Number of hemisphere pixels
$m_a$: Mask of hemisphere a
t: phase time point
G(): Intensity transformation function e.g., normalization or thresholding

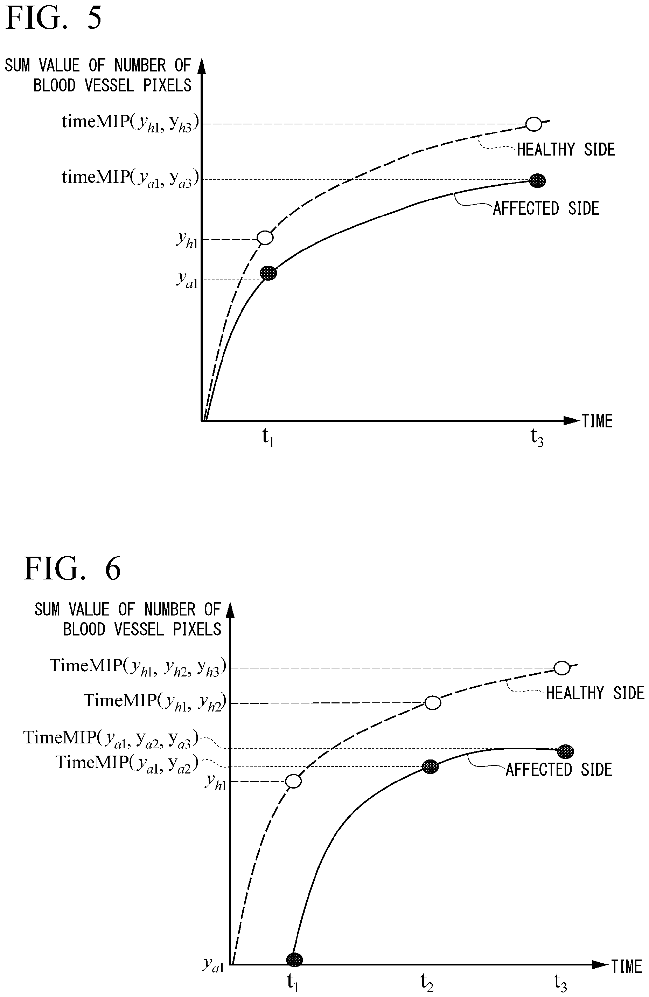

Alternatively, the derivation function 144 calculates the left/right presence ratio Output(2) at a specific timing by putting the number of blood vessel pixels in a plurality of time phases into the following formula (3). Change over time in the sum value of the number of blood vessel pixels on each of the affected side and the healthy side at this time is shown as a graph in FIG. 5. FIG. 5 is a diagram showing an example of change over time in the sum value of the number of blood vessel pixels on each of the affected side and the healthy side.

[Expression 2]

$$\text{Output}(2) = \frac{ya1/TimeMIP(ya1,ya3)}{yh1/TimeMIP(yh1,yh3)} \quad \cdots (3)$$

ya1: Number of affected-side blood vessel pixels (time t1)
TimeMIP(ya1,ya3): Sum value of number of affected-side blood vessel pixels (time t1 and t3)
yh1: Number of healthy-side blood vessel pixels (time t1)
TimeMIP(yh1,yh3): Sum value of number of healthy-side blood vessel pixels (time t1 and t3)

Alternatively, the derivation function 144 calculates the left/right outflow ratio Output(3) in a plurality of time phases by putting the number of blood vessel pixels in the plurality of time phases into the following formula (4). Change over time in the sum value of the number of blood vessel pixels on each of the affected side and the healthy side at this time is shown as a graph in FIG. 6. FIG. 6 is a diagram showing an example of change over time in the sum value of the number of blood vessel pixels on each of the affected side and the healthy side.

[Expression 3]

$$\text{Output}(3) = \frac{TimeMIP(ya1,ya2,ya3) - TimeMIP(ya1,ya2) - ya1}{TimeMIP(yh1,yh2,yh3) - TimeMIP(yh1,yh2) - yh1} \quad \cdots (4)$$

ya1: Number of affected-side blood vessel pixels (time t1)
TimeMIP(ya1,ya2): Sum value of number of affected-side blood vessel pixels (time t1 and t2)
TimeMIP(ya1,ya2,ya3): Sum value of number of affected-side blood vessel pixels (time t1, t2, and t3)
yh1: Number of healthy-side blood vessel pixels (time t1)
TimeMIP(yh1,yh2): Sum value of number of healthy-side blood vessel pixels (time t1 and t2)
TimeMIP(yh1,yh2,yh3): Sum value of number of healthy-side blood vessel pixels (time t1, t2, and t3)

Subsequently, the analysis function 145 analyzes a time phase delay in the affected side relative to the healthy side on the basis of the left-right ratio derived by the derivation function 144. For example, when the derivation function 144 derives the left-right inflow ratio Output(1), the analysis function 145 compares the left-right inflow ratio Output(1) with the first threshold value (here, 0.5). When Output(1) $\geq$ 0.5, the analysis function 145 determines that there is no time phase delay, and when Output(1) < 0.5, the analysis function 145 determines that there is a time phase delay. The first threshold value may be a value other than "0.5."

For example, when the derivation function 144 derives the left-right presence ratio Output(2), the analysis function 145 compares the left-right presence ratio Output(2) with the second threshold value (here, 0.5). Here, the left/right presence ratio Output(2) compares presence or absence of a delay between a first time phase (=11) and a third time phase (=t3). When Output(2) $\geq$ 0.5, the analysis function 145 determines that there is no phase delay ($\alpha$ phase delay) between the first time phase (=11) and the third time phase (=t3) being compared, and when Output(2) < 0.5, the analysis function 145 determines that there is a phase delay. The second threshold value may be a value other than "0.5." The second threshold value is the same value as the first threshold value, but the second threshold value may be different from the first threshold value.

For example, when the derivation function 144 derives the left/right outflow ratio Output(3), the analysis function 145 compares the left/right outflow ratio Output(3) with the third threshold value (here, 0.5). When Output(3) $\geq$ 0.5, the analysis function 145 determines that the amount of fluid flowing out from collateral circulation is equal between the affected side and the healthy side in the plurality of time phases and that there is no phase delay. When Output(3) < 0.5, the analysis function 145 determines that the amount of fluid flowing out from collateral circulation is less on the affected side than on the healthy side in the plurality of time phases and that there is a phase delay. The third threshold value may be a value other than "0.5." The third threshold value is the same as the first threshold value, but the third threshold value may be different from the first threshold value.

After the analysis of the left-right ratio by the analysis function 145 is completed, the display control function 146 causes the display 130 to display the MIP images, blood vessel images, the analysis results, and the like. The display control function 146 displays the MIP images, blood vessel images, and the analysis results as appropriate, and may not display some or all of the same.

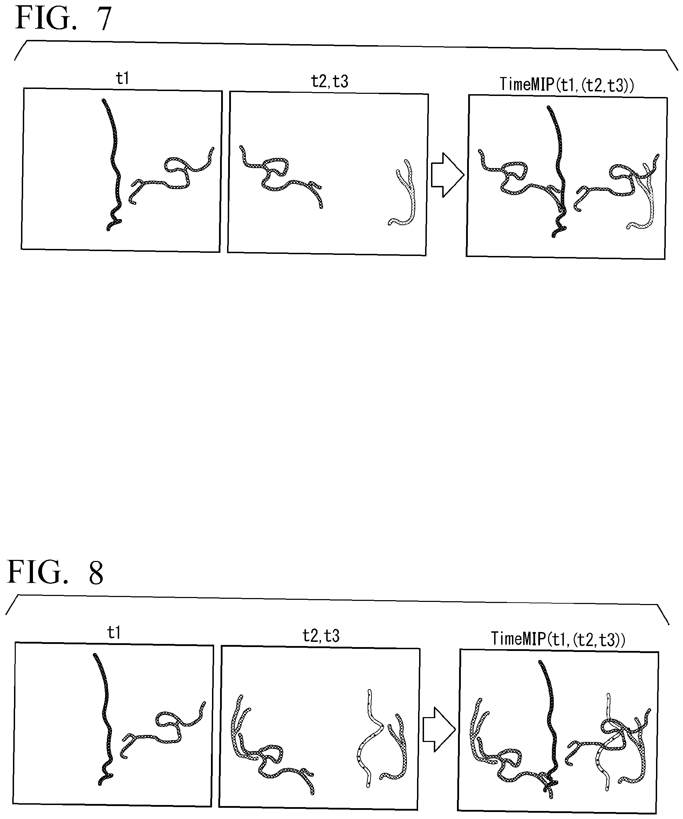

The generation function 143 may generate a combined MIP image of an n-th time phase and an (n+$\alpha$)-th time phase, for example, and the display control function 146 may cause the display 130 to display the generated combined MIP image. FIG. 7 is a diagram showing an example of images of a procedure for combining images for two time phases. The generation function 143 may generate a combined MIP image TimeMIP(t1, t2) by combining an MIP image of the first time phase (=t1) with an MIP image of the second time phase (=t2), for example, and the display control function 146 may cause the display 130 to display the generated combined MIP image TimeMIP(t1, t2).

Similarly, the generation function 143 may generate a combined MIP image of the first time phase and the third time phase, or may generate a combined MIP image of the second time phase and the third time phase. The generation function 143 may generate a blood vessel image on the basis of the combined MIP image, and the display control function 146 may cause the display 130 to display the generated blood vessel image.

For example, the generation function 143 may generate an MIP image by combining images for one time phase and a plurality of time phases (e.g., two time phases). FIG. 8 is a diagram showing an example of images of a procedure for combining images for one time phase and two time phase images. For example, the generation function 143 may generate a combined MIP image TimeMIP(t1, (t2, t3)) by combining an MIP image of the first time phase (=t1) with two time phase images t1 and t2 for the second time phase (=t2) and third time phase (=t3).

Similarly, the generation function 143 may generate a combined MIP image by combining the second phase with images for two phases of the first and third phases, or may generate a combined MIP image by combining the third phase with images for two phases of the first and third phases. The generation function 143 may further generate a vascular image on the basis of the combined MIP image, and the display control function 146 may cause the display 130 to display the generated vascular image. In this manner, the medical image processing device 100 ends the processing shown in FIG. 3.

In the first embodiment, the medical image processing device 100 compares feature values of the affected side and the healthy side derived for each of a plurality of time phases on the basis of four-dimensional CT images captured by the modality 30 with a threshold value to analyze a delay of the affected side relative to the healthy side. This allows the user to accurately diagnose the condition of collateral circulation. Since collateral circulation is a factor that affects the prognosis of a patient, feature values such as the left-right ratio can be used as an index for treatment decision.

(Second embodiment)

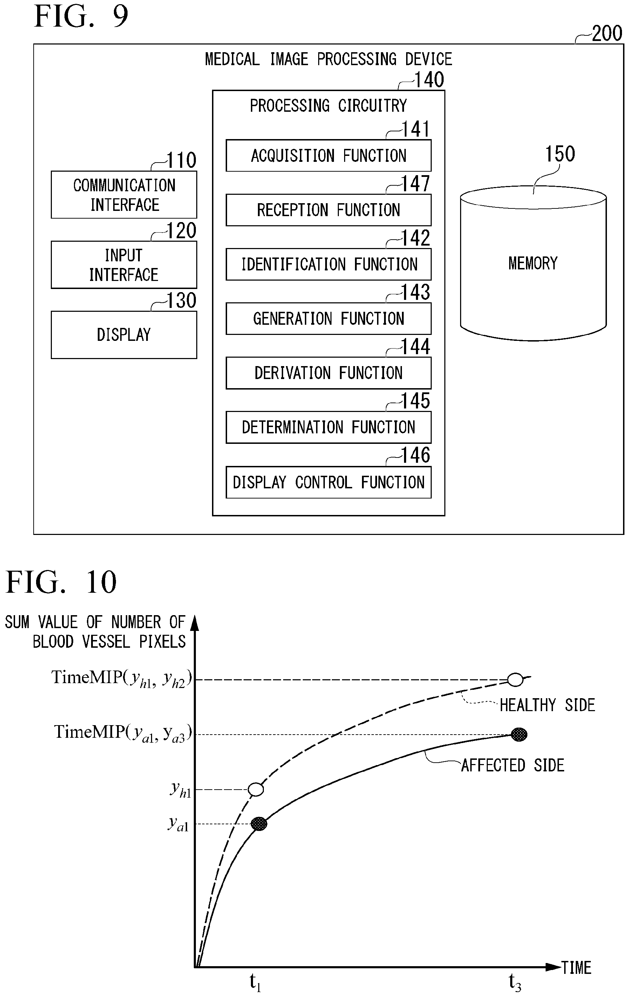

Next, a second embodiment will be described. FIG. 9 is a block diagram showing an example of a configuration of a medical image processing device 200 of the second embodiment. The medical image processing device 200 of the second embodiment differs mainly from the medical image processing device 100 of the first embodiment in the configuration of the processing circuitry 140. Hereinafter, the second embodiment will be described, focusing on the differences from the first embodiment. In the following description, the same reference numerals may be used for common elements, and description thereof may be omitted.

The processing circuitry 140 in the medical image processing device 200 of the second embodiment includes a reception function 147 in addition to the acquisition function 141, the identification function 142, the generation function 143, the derivation function 144, the analysis function 145, and the display control function 146. The other functions are common to the medical image processing device 100 of the first embodiment.

The reception function 147 receives identification of an MIP range by the user. The reception function 147 receives identification information transmitted through the input interface 120 by the user operating the input interface 120, for example. The identification information is information for identifying an MIP range in a four-dimensional CT image. The reception function 147 is an example of a receiver.

The identification function 142 identifies an MIP range in the four-dimensional CT image on the basis of the identification information received by the reception function 147. In identifying the MIP range, the identification function 142 divides the four-dimensional CT image acquired by the acquisition function 141 into an affected side and a healthy side. The identification function 142 identifies the MIP range on each of the affected side and the healthy side. The identification function 142 may identify the MIP range on the basis of information other than information in a case where the identification information is received, for example, information on the location where the infarction is assumed to have occurred. The identification function 142 is an example of an identifier.

The medical image processing device 200 of the second embodiment has the same effect as the medical image processing device 100 of the first embodiment. Furthermore, in the medical image processing device 200 of the second embodiment, the reception function 147 receives identification of the MIP range by the user. Therefore, the state of cerebral infarction in the MIP range according to a user request can be diagnosed with high accuracy. For example, the user can designate the range of only the MCA region where collateral circulation is particularly developed.

In each of the above embodiments, the derivation function 144 derives the left-right inflow ratio, the left-right presence ratio, and the left-right outflow ratio as feature amounts on the affected side and healthy side. On the other hand, the derivation function 144 may generate a side-specific collateral circulation time ratio that compares the presence ratio of blood vessels between different time phases on the affected side and healthy side. Furthermore, the analysis function 145 may determine a time phase delay on the basis of the derived side-specific collateral circulation time ratio on the affected side and healthy side.

The derivation function 144 calculates an affected-side collateral circulation time ratio Output (4), which is the side-specific collateral circulation time on the affected side, by putting the number of blood vessel pixels on the affected side in a plurality of time phases into the following formula (5). Change over time in the sum value of the number of blood vessel pixels on each of the affected side and the healthy side at this time is shown as a graph in FIG. 10. FIG. 10 is a diagram showing an example of change over time in the sum value of the number of blood vessel pixels on each of the affected side and the healthy side.

[Expression 4]

$$Output(4) = \frac{ya1}{TimeMIP(ya1, ya3)} \quad \dots (4)$$

ya1: Number of affected-side blood vessel pixels (time t1)
TimeMIP(ya1,ya3): Sum value of number of affected-side blood vessel pixels (time t1 and t3)

The analysis function 145 analyzes a delay in the time phase of the affected side relative to the healthy side on the basis of the affected-side collateral circulation time ratio Output(4) derived by the derivation function 144. The analysis function 145 can obtain the amount of change in blood per unit time, for example, when the affected-side collateral circulation time ratio Output(4), which is the ratio of the number of blood vessel pixels in the first time phase to the sum value of the number of blood vessel pixels in the first and third time phases, is processed with a certain threshold value. It may be determined that there is a time phase delay when the amount of change in blood is, for example, equal to or less than a predetermined threshold value.

In each of the above embodiments, the analysis function 145 may also analyze a time phase delay on the basis of the amount of change over time in the total filling amount on the affected side. The analysis function 145 may obtain the amount of change in the volume of the fluid (contrast agent or blood) in the brain, Output(5), using the following formula (6), for example, as change over time in the total filling amount on the affected side, and determine a time phase delay on the basis of the amount of change in the volume of the fluid, Output(5). In this case, for example, a threshold value, for example, $25\,mm^3$, may be set for the amount of change, and it may be determined that there is a time phase delay when the amount of change, Output(5), is equal to or less than the threshold value.

[Expression 5]

$$\text{Output}(5) = \frac{ya1}{TimeMIP(ya1,ya3)} \div (t3 - t1) \quad \dots (6)$$

ya1: Number of affected-side blood vessel pixels (time t1)
TimeMIP(ya1,ya3): Sum value of number of affected-side blood vessel pixels (time t1 and t3)

According to at least one of the embodiments described above, the medical image processing device can evaluate the condition of a disease with high accuracy by including the acquirer that acquires original medical images of the brain of a subject in a plurality of time phases, having one of left and right sides of the brain as an affected side and the other as a healthy side, the deriver that derives feature amounts related to collateral circulation in the brain of the subject on the basis of the acquired original medical images, and the analyzer that analyzes a time phase delay in the affected side relative to the healthy side on the basis of a result of comparing the feature amounts of the affected side and the healthy side for each of the plurality of time phases.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A medical image processing device comprising:

   an acquirer configured to acquire original medical images of a plurality of time phases of a brain of a subject, having one of left and right sides of the brain as an affected side and the other as a healthy side;
   a deriver configured to derive feature amounts related to collateral circulation in the brain of the subject based on the acquired original medical images; and
   an analyzer configured to analyze a time phase delay in the affected side relative to the healthy side based on a result of comparing the feature amounts of the affected side and the healthy side for each of the plurality of time phases.

2. The medical image processing device according to claim 1, further comprising a display controller configured to cause a display to display delay information regarding the determined time phase delay.

3. The medical image processing device according to claim 1, wherein the deriver is configured to derive a ratio between inflow amounts of fluid flowing into blood vessels on the affected side and the healthy side for each of the plurality of time phases, and the analyzer is configured to analyze the time phase delay based on the ratio between the inflow amounts.

4. The medical image processing device according to claim 1, wherein the deriver is configured to derive a ratio between presence of blood vessels on the affected side and the healthy side for each of the plurality of time phases, and the analyzer is configured to analyze the time phase delay based on the ratio between the presence.

5. The medical image processing device according to claim 1, wherein the deriver is configured to derive a ratio between outflow amounts of fluid flowing out of blood vessels on the affected side and the healthy side for each of the plurality of time phases, and the analyzer is configured to analyze the time phase delay based on the ratio between the outflow amounts.

6. The medical image processing device according to claim 3, wherein the analyzer is configured to determine that there is a time phase delay when the ratio between the inflow amounts is less than a first threshold value.

7. The medical image processing device according to claim 4, wherein the analyzer is configured to determine that there is the time phase delay when the ratio between the presence is less than a second threshold value.

8. The medical image processing device according to claim 5, wherein the analyzer is configured to determine that there is a time phase delay when the ratio between the outflow amounts is equal to or greater than a third threshold value.

9. The medical image processing device according to claim 1, wherein the deriver is configured to derive an affected-side collateral circulation time ratio by comparing blood vessel presence ratios in different time phases on the affected side, and
the analyzer is configured to determine the time phase delay based on the affected-side collateral circulation time ratio.

10. The medical image processing device according to claim 1, further comprising a generator configured to perform maximum intensity projection processing on the original medical images to generate a maximum intensity projection image,
wherein the deriver is configured to derive the feature amounts based on the maximum intensity projection image.

11. The medical image processing device according to claim 10, further comprising an identifier configured to identify a projection processing range that is a target of the maximum intensity projection processing.

12. The medical image processing device according to claim 11, further comprising a receiver configured to receive identification of the projection processing range by a user.

13. The medical image processing device according to claim 1, wherein the analyzer is configured to analyze the time phase delay based on an amount of change over time in a total vascular filling amount on the affected side.

14. A medical image processing method, using a computer, comprising:

acquiring original medical images of a plurality of time phases of a brain of a subject, with one of left and right sides of the brain being an affected side and the other being a healthy side;
deriving feature amounts related to collateral circulation in the brain of the subject based on the acquired original medical images; and
analyzing a time phase delay in the affected side relative to the healthy side based on a result of comparing the feature amounts of the affected side and the healthy side for each of the plurality of time phases.

15. A program causing a computer to:

acquire original medical images of a plurality of time phases of a brain of a subject, with one of left and right sides of the brain being an affected side and the other being a healthy side;
derive feature amounts related to collateral circulation in the brain of the subject based on the acquired original medical images; and
analyze a time phase delay in the affected side relative to the healthy side based on a result of comparing the feature amounts of the affected side and the healthy side for each of the plurality of time phases.

# FIG. 1

MEDICAL IMAGE
PROCESSING DEVICE ~100

1

10~ HIS

40~ PACS

NW

20~ RIS

30~ MODALITY

# FIG. 2

~100

MEDICAL IMAGE PROCESSING DEVICE

PROCESSING CIRCUITRY ~140

~141 ACQUISITION FUNCTION

~110 COMMUNICATION INTERFACE

~142 IDENTIFICATION FUNCTION

~120 INPUT INTERFACE

~143 GENERATION FUNCTION

150 MEMORY

~130 DISPLAY

~144 DERIVATION FUNCTION

~145 ANALYSIS FUNCTION

~146 DISPLAY CONTROL FUNCTION

# FIG. 3

```
        START
          │
          ▼                    S101
┌─────────────────────────────┐
│ ACQUIRE FOUR-DIMENSIONAL CT IMAGE │
└─────────────────────────────┘
          │
          ▼                    S103
┌─────────────────────────────┐
│      IDENTIFY MIP RANGE      │
└─────────────────────────────┘
          │
          ▼                    S105
┌─────────────────────────────┐
│ GENERATE MIP IMAGE OF EACH TIME PHASE │
└─────────────────────────────┘
          │
          ▼                    S107
┌─────────────────────────────┐
│    GENERATE BLOOD VESSEL IMAGE    │
└─────────────────────────────┘
          │
          ▼                    S109
┌─────────────────────────────┐
│     ANALYZE LEFT-RIGHT RATIO     │
└─────────────────────────────┘
          │
          ▼
        END
```

# FIG. 4

SUM VALUE OF NUMBER OF BLOOD VESSEL PIXELS

$\text{TimeMIP}(y_{h1}, y_{h2}, y_{h3})$

$\text{TimeMIP}(y_{h1}, y_{h2})$

HEALTHY SIDE

$\text{TimeMIP}(y_{a1}, y_{a2}, y_{a3})$

$\text{TimeMIP}(y_{a1}, y_{a2})$

$y_{h1}$

AFFECTED SIDE

$y_{a1}$

$t_1$　　$t_2$　　$t_3$　　TIME

# FIG. 5

SUM VALUE OF NUMBER OF BLOOD VESSEL PIXELS

$\text{timeMIP}(y_{h1}, y_{h3})$

$\text{timeMIP}(y_{a1}, y_{a3})$

HEALTHY SIDE

AFFECTED SIDE

$y_{h1}$

$y_{a1}$

$t_1$

$t_3$

TIME

# FIG. 6

SUM VALUE OF NUMBER OF BLOOD VESSEL PIXELS

$\text{TimeMIP}(y_{h1}, y_{h2}, y_{h3})$

$\text{TimeMIP}(y_{h1}, y_{h2})$

HEALTHY SIDE

$\text{TimeMIP}(y_{a1}, y_{a2}, y_{a3})$

$\text{TimeMIP}(y_{a1}, y_{a2})$

$y_{h1}$

AFFECTED SIDE

$y_{a1}$

$t_1$

$t_2$

$t_3$

TIME

## FIG. 7

t1　　　　　　　t2,t3　　　　　　TimeMIP(t1,(t2,t3))

## FIG. 8

t1　　　　　　　t2,t3　　　　　　TimeMIP(t1,(t2,t3))

## FIG. 9

~200

**MEDICAL IMAGE PROCESSING DEVICE**

~140

**PROCESSING CIRCUITRY**

~141
ACQUISITION FUNCTION

~147
RECEPTION FUNCTION

~142
IDENTIFICATION FUNCTION

~143
GENERATION FUNCTION

~144
DERIVATION FUNCTION

~145
DETERMINATION FUNCTION

~146
DISPLAY CONTROL FUNCTION

~110
COMMUNICATION INTERFACE

~120
INPUT INTERFACE

~130
DISPLAY

150
MEMORY

## FIG. 10

SUM VALUE OF NUMBER OF BLOOD VESSEL PIXELS

$\text{TimeMIP}(y_{h1}, y_{h2})$

$\text{TimeMIP}(y_{a1}, y_{a3})$

$y_{h1}$

$y_{a1}$

HEALTHY SIDE

AFFECTED SIDE

$t_1$

$t_3$

TIME

Europäisches
Patentamt

European
Patent Office

Office européen
des brevets

## EUROPEAN SEARCH REPORT

**Application Number**

EP 25 15 4820

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARTA KERSTEN-OERTEL ET AL: "Towards a Computed Collateral Circulation Score in Ischemic Stroke", ARXIV.ORG, CORNELL UNIVERSITY LIBRARY, 201 OLIN LIBRARY CORNELL UNIVERSITY ITHACA, NY 14853, 20 January 2020 (2020-01-20), XP081582385, | 1,2, 10-15 | INV. A61B6/03 A61B6/00 A61B6/50 |
| Y | * the whole document * | 3-8 | |
| A | | 9 | |
| | ----- | | |
| X | SINGH NISHITA ET AL: "Time-resolved assessment of cortical venous drainage on multiphase CT angiography in patients with acute ischemic stroke", NEURORADIOLOGY, SPRINGER BERLIN HEIDELBERG, BERLIN/HEIDELBERG, vol. 64, no. 5, 26 October 2021 (2021-10-26), pages 897-903, XP037811700, ISSN: 0028-3940, DOI: 10.1007/S00234-021-02837-1 [retrieved on 2021-10-26] | 1,2, 10-15 | |
| Y | * page 898; figure 1 * | 3-8 | TECHNICAL FIELDS SEARCHED (IPC) A61B |
| A | | 9 | |
| | ----- | | |
| | -/-- | | |

The present search report has been drawn up for all claims

| Place of search | Date of completion of the search | Examiner |
|---|---|---|
| Munich | 16 June 2025 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 595 891 A1**

| | Europäisches Patentamt<br>European Patent Office<br>Office européen des brevets | **EUROPEAN SEARCH REPORT** | **Application Number**<br>EP 25 15 4820 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WU XIAOLING ET AL: "Ultra-low-dose multiphase CT angiography derived from CT perfusion data in patients with middle cerebral artery stenosis",<br>NEURORADIOLOGY, SPRINGER BERLIN HEIDELBERG, BERLIN/HEIDELBERG,<br>vol. 62, no. 2,<br>30 October 2019 (2019-10-30), pages 167-174, XP036992980,<br>ISSN: 0028-3940, DOI:<br>10.1007/S00234-019-02313-X<br>[retrieved on 2019-10-30] | 1,2,<br>10-15 | |
| Y | * fig. 2, (f), (g), (h); | 3-8 | |
| A | page 172; figure 2 * | 9 | |
| X | OSPEL JOHANNA M ET AL: "Utility of Time-Variant Multiphase CTA Color Maps in Outcome Prediction for Acute Ischemic Stroke Due to Anterior Circulation Large Vessel Occlusion",<br>CLINICAL NEURORADIOLOGY, SPRINGER BERLIN HEIDELBERG, BERLIN/HEIDELBERG,<br>vol. 31, no. 3,<br>25 September 2020 (2020-09-25), pages 783-790, XP037571652,<br>ISSN: 1869-1439, DOI:<br>10.1007/S00062-020-00958-3<br>[retrieved on 2020-09-25] | 1,2,<br>10-15 | TECHNICAL FIELDS<br>SEARCHED (IPC) |
| Y | * page 786 * | 3-8 | |
| A | | 9 | |

-/--

The present search report has been drawn up for all claims

| Place of search | Date of completion of the search | Examiner |
|---|---|---|
| Munich | 16 June 2025 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

18

Europäisches
Patentamt

European
Patent Office

Office européen
des brevets

## EUROPEAN SEARCH REPORT

**Application Number**

EP 25 15 4820

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LU SHAN-SHAN ET AL: "Comparison of CT angiography collaterals for predicting target perfusion profile and clinical outcome in patients with acute ischemic stroke", EUROPEAN RADIOLOGY, SPRINGER BERLIN HEIDELBERG, BERLIN/HEIDELBERG, vol. 29, no. 9, 14 February 2019 (2019-02-14), pages 4922-4929, XP036852181, ISSN: 0938-7994, DOI: 10.1007/S00330-019-06027-9 [retrieved on 2019-02-14] | 1,2, 10-15 | |
| Y | * page 4924 * | 3-8 | |
| A | | 9 | |
| | ----- | | |
| X | US 2016/157800 A1 (GOYAL MAYANK [CA]) 9 June 2016 (2016-06-09) | 1,2, 10-15 | |
| Y | * paragraphs [0171], [0180]; tables 4,6 * | 3-8 | |
| A | | 9 | |
| | ----- | | TECHNICAL FIELDS SEARCHED (IPC) |
| X | JP 2005 095340 A (TOSHIBA CORP) 14 April 2005 (2005-04-14) | 1,2, 10-15 | |
| Y | * paragraph [0086] * | 3-8 | |
| A | | 9 | |
| | ----- | | |

The present search report has been drawn up for all claims

| Place of search | Date of completion of the search | Examiner |
|---|---|---|
| Munich | 16 June 2025 | Anscombe, Marcel |

EPO FORM 1503 03.82 (P04C01)

page 3 of 3

**ANNEX TO THE EUROPEAN SEARCH REPORT
ON EUROPEAN PATENT APPLICATION NO.**

EP 25 15 4820

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2025

| Patent document cited in search report | Publication date | Patent family member(s) | | Publication date |
|---|---|---|---|---|
| US 2016157800 A1 | 09-06-2016 | NONE | | |
| JP 2005095340 A | 14-04-2005 | CN | 1600268 A | 30-03-2005 |
| | | JP | 4537681 B2 | 01-09-2010 |
| | | JP | 2005095340 A | 14-04-2005 |
| | | US | 2005065432 A1 | 24-03-2005 |

EPO FORM P0459

For more details about this annex : see Official Journal of the European Patent Office, No. 12/82